## Europäisches Patentamt
## European Patent Office
## Office européen des brevets

(19)

(11) Veröffentlichungsnummer: **0 138 151 B1**

# EUROPÄISCHE PATENTSCHRIFT

(45) Veröffentlichungstag der Patentschrift: **28.08.91**

(21) Anmeldenummer: **84111799.7**

(22) Anmeldetag: **03.10.84**

(51) Int. Cl.⁵: **C07D 223/04**, C07D 201/12, C07D 223/10

(54) **Verfahren zur Herstellung von N-C1-C4-Alkylcaprolactam.**

(30) Priorität: 05.10.83 DE 3336142

(43) Veröffentlichungstag der Anmeldung:
24.04.85 Patentblatt 85/17

(45) Bekanntmachung des Hinweises auf die
Patenterteilung:
28.08.91 Patentblatt 91/35

(84) Benannte Vertragsstaaten:
**BE CH DE IT LI NL**

(56) Entgegenhaltungen:
**EP-A- 0 046 183**
**DD-A- 96 483**

**CHEMICAL ABSTRACTS, Band 95, Nr.15, 12
Oktober 1981 Columbus, OH (US); K.WEHNER
et al., Seite 617, Nr.132263j**

**CHEMICAL ABSTRACTS, Band 91, Nr.8, 20
August 1979, Columbus, OH (US); Seite 19,
Nr.57799a, Nr.57800u**

(73) Patentinhaber: **BASF Aktiengesellschaft
Carl-Bosch-Strasse 38
W-6700 Ludwigshafen(DE)**

(72) Erfinder: **Fuchs, Hugo, Dr.
Egellstr. 28
W-6700 Ludwigshafen(DE)**
Erfinder: **Grosskinsky, Otto-Alfred, Dr.
Semmelweissstr. 8
W-6700 Ludwigshafen(DE)**
Erfinder: **Frommer, Elmar, Dr.
Lisztstr. 117
W-6700 Ludwigshafen(DE)**

## Beschreibung

Bei der Herstellung von Caprolactam erhält man zunächst Polycaprolactam mit einem Gehalt an Monomeren und Oligomeren von ca. 10 Gew.%. Aus dem Monomeren enthaltenden Polycaprolactam werden die Monomeren und die Oligomeren nach bekannten Verfahren z.B. durch Wasser extrahiert. Nach Abdampfen des Wassers wird das extrahierte Caprolactam zurückgewonnen. Es verbleiben jedoch noch erhebliche Mengen an Oligomeren, die behandlungsbedürftig sind.

Aus der DD-PS 96 483 ist ein Verfahren zur Herstellung von N-Methylcaprolactam bekannt, bei dem man $\epsilon$-Caprolactam zusammen mit Methanol bei einer Temperatur von 350°C über einen fest angeordneten Aluminiumoxidkatalysator leitet. $\epsilon$-Caprolactam ist jedoch ein relativ wertvolles Ausgangsprodukt.

Es war deshalb die technische Aufgabe gestellt, N-$C_1$-$C_4$-Alkylcaprolactam, ausgehend von einem wohlfeileren Ausgangsprodukt herzustellen und wenig verwertbare Oligomere des Caprolactams in Wertprodukte zu überführen.

Diese Aufgabe wird gelost in einem Verfahren zur Herstellung von N-$C_1$-$C_4$-Alkylcaprolactam, bei dem man Oligomere des Caprolactams mit einem Polymerisationsgrad von 2 bis 9 in einer Aluminiumoxidwirbelschicht mit $C_1$-$C_4$-Alkanolen in Gegenwart von Wasserdampf bei einer Temperatur von 290 bis 400°C umsetzt.

Das neue Verfahren hat den Vorteil, daß man anstatt von Caprolactam von wohlfeilen Oligomeren des Caprolactams ausgeht. Das neue Verfahren ist insofern bemerkenswert als nicht zu erwarten war, daß sich Oligomere des Caprolactams ohne größere Schwierigkeiten zu N-Alkylcaprolactam umsetzen lassen, da in Chem. Techn., 33. Jg., Heft 4, 1981, S. 194 beschrieben wird, daß bei der Umsetzung von Caprolactam mit Methanol durch Ringspaltungsreaktionen, die zweifellos bei der Oligomerenspaltung eintreten, Folgeumsetzungen der Bruchstücke eintreten und zudem Kondensationsreaktionen zu teerartigen Produkten führen.

Oligomere des Caprolactams, die als Ausgangsstoffe eingesetzt werden, haben einen Polymerisationsgrad n von 2 bis 9. Insbesondere enthalten sie dimere und trimere cyclische Oligomere. Solche Oligomere werden beispielsweise erhalten durch Eindampfen von Waschwässern, die bei der Extraktion von Polycaprolactam anfallen und anschließende Entfernung des monomeren Caprolactams durch Destillation. Es ist jedoch nicht erforderlich aus dem anfallenden Extrakt die gesamte Caprolactammenge abzudestillieren. Die Oligomeren, gegebenenfalls mit Restmengen an Caprolactam, werden vorteilhaft in flüssiger Form, d.h. in geschmolzenem Zustand z.B. bei einer Temperatur von 150 bis 250°C in eine als Katalysator wirkende Aluminiumoxidwirbelschicht eingebracht. Es ist jedoch auch möglich, die Oligomeren in fester, feinverteilter Form in die Wirbelschicht einzubringen. Das Einbringen in die Wirbelschicht erfolgt z.B. durch Einblasen mittels einer mit Inertgas betriebenen Düse.

Als Aluminiumoxid eignen sich die verschiedenen Modifikationen wie Tonerde, Böhmit oder $\gamma$-Aluminiumoxid. Besonders bewährt hat sich $\gamma$-Aluminiumoxid als Katalysator. Der Katalysator wird mit Inertgas wie Kohlendioxid, Argon oder Stickstoff, vorzugsweise Stickstoff, in wirbelnder Bewegung gehalten. Zweckmäßig verwendet man Aluminiumoxid in Korngrößen von 0,05 bis 1,5 mm, insbesondere von 0,2 bis 1 mm. Die Höhe der Wirbelschicht wird vorteilhaft so gewählt, daß die Verweilzeiten der Oligomeren an der Katalysatorschicht von 0,1 bis 30, insbesondere von 0,5 bis 10 sec. betragen. Vorteilhaft führt man das Verfahren bei Normaldruck durch. Es kann jedoch auch bei schwach vermindertem oder leichtem Überdruck, z.B. bis zu 2 bar durchgeführt werden.

Die Wirbelschicht wird auf einer Temperatur von 290 bis 400°C, insbesondere 300 bis 360°C gehalten. Es ist deshalb auch zweckmäßig, das Inertgas mit einer Temperatur von 290 bis 400°C der Wirbelschicht zuzuführen.

Die Umsetzung wird mit $C_1$-$C_4$-Alkanolen durchgeführt. Geeignete Alkanole sind beispielsweise Methanol, Ethanol, Propanol, Butanol oder Isopropanol. Besonders bevorzugt wird Methanol verwendet. Demzufolge ist das bevorzugte Endprodukt auch N-Methylcaprolactam. Vorteilhaft wendet man je Gewichtsteil an Oligomeren 0,3 bis 10 Gewichtsteile Alkanole an. Die Alkanole können als solche in die Wirbelschicht eingebracht und dort verdampft werden. Vorzugsweise führt man die Alkanole jedoch in Dampfform, z.B. zusammen mit dem Intertgas, der Wirbelschicht zu.

Erfindungsgemäß erfolgt die Umsetzung unter Mitverwendung von Wasserdampf. Vorteilhaft verwendet man je Gewichtsteil Oligomere 0,005 bis 10 Gew.teile, insbesondere 0,02 bis 2 Gew.teile Wasser in Form von Wasserdampf. Wasser kann wie die Alkanole in die Wirbelschicht eingebracht werden.

Aus dem aus der Wirbelschicht austretenden Gasgemisch werden die kondensierbaren Anteile abgeschieden und durch Destillation aufgearbeitet. Bei der Umsetzung entstehendes Caprolactam kann wieder in die Wirbelschicht zurückgeführt werden. Ebenso kann überschüssiges Alkanol separat oder zusammen mit dem kondensierten Wasser in den Kreislauf zurückgeführt werden. Des Wirbelgas kann im Kreis geführt werden.

N-$C_1$-$C_4$-Alkylcaprolactam eignet sich als selektives Lösungsmittel bei petrochemischen Verfahren.

Das Verfahren nach der Erfindung sei an folgendem Beispiel veranschaulicht.

Beispiel

In einem senkrecht stehenden isolierten elektrisch beheizten, unten mit einem Lochboden versehenen Rohr von 900 mm Länge und 50 mm Durchmesser werden 420 g Katalysator, bestehend aus einem bei 800° C geglühten γ-Aluminiumoxid, Korngröße 0,2 bis 0,8 mm, auf 360° C erhitzt. Durch einen von unten durch den Lochboden eingeblasenen, auf 360° C vorgewärmten Stickstoffstrom von 400 1/h, wird der Katalysator zum Wirbeln gebracht. Dem vorgeheizten Stickstoffstrom werden innerhalb von 2 Stunden 253 g Methanol und 253 g Wasser zudosiert. Gleichzeitig wird durch eine Düse oberhalb des Lochbodens, die sich zentrisch im Rohr befindet, mit Hilfe eines Stickstoffstroms, der vorgeheizt ist, innerhalb von 2 Stunden 300 g oligomeres Caprolactam zugeführt. Die den Reaktor verlassenden Dämpfe werden kondensiert und das Kondensat durch Destillation aufgearbeitet. Man erhält 122,5 g N-Methylcaprolactam, sowie 174 g monomeres Caprolactam.

**Patentansprüche**

1. Verfahren zur Herstellung von N-C$_1$-C$_4$-Alkylcaprolactam, dadurch gekennzeichnet, daß man Oligomere des Caprolactams mit einem Polymerisationsgrad von 2 bis 9 in einer Aluminiumoxidwirbelschicht mit C$_1$-C$_4$-Alkanolen in Gegenwart von Wasserdampf bei einer Temperatur von 290 bis 400° C umsetzt.

2. Verfahren nach Anspruch 1, dadurch gekennzeichnet, daß man je Gewichtsteil Oligomere 0,3 bis 10 Gewichtsteile Alkanole anwendet.

3. Verfahren nach den Ansprüchen 1 und 2, dadurch gekennzeichnet, daß man je Gewichtsteil Oligomere 0,005 bis 10 Gewichtsteile Wasser in Form von Wasserdampf mitverwendet.

4. Verfahren nach den Ansprüchen 1 bis 3, dadurch gekennzeichnet, daß man die Oligomeren flüssig oder in festem Zustand in die Aluminiumoxidwirbelschicht einbringt.

5. Verfahren nach den Ansprüchen 1 bis 4, dadurch gekennzeichnet, daß man γ-Aluminiumoxid für die Aluminiumoxidwirbelschicht verwendet.

6. Verfahren nach den Ansprüchen 1 bis 5, dadurch gekennzeichnet, daß man als Alkanol Methanol verwendet.

**Claims**

1. A process for the preparation of an N-C$_1$-C$_4$-alkylcaprolactam, wherein an oligomer of caprolactam having a degree of polymerization of from 2 to 9 is reacted with a C$_1$-C$_4$-alkanol in a fluidized aluminum oxide bed in the presence of steam at from 290 to 400° C.

2. A process as claimed in claim 1, wherein from 0.3 to 10 parts by weight of an alkanol are used per part by weight of oligomer.

3. A process as claimed in either of claims 1 and 2, wherein from 0.005 to 10 parts by weight of water in the form of steam are present per part by weight of oligomer.

4. A process as claimed in any of claims 1 to 3, wherein the oligomer is introduced in liquid or solid form into the fluidized aluminum oxide bed.

5. A process as claimed in any of claims 1 to 4, wherein γ-aluminum oxide is used for the fluidized bed.

6. A process as claimed in any of claims 1 to 5, wherein methanol is used as the alkanol.

**Revendications**

1. Procédé de préparation de N-alkyle en C$_1$ à c$_4$-caprolactame, caractérisé en ce qu'on convertit des oligomères de caprolactame ayant un degré de polymérisation de 2 à 9 dans un lit fluidisé d'oxyde d'aluminium avec des alcanols en c$_1$ à c$_4$ en présence de vapeur d'eau à une température de 290 à 400° C.

2. Procédé selon la revendication 1, caractérisé en ce qu'on utilise de 0,3 à 10 parties en poids d'alcanols par partie en poids d'oligoméres.

3. Procédé selon les revendications 1 et 2, caractérisé en ce qu'on utilise de 0,005 à 10 parties en poids d'eau sous forme de vapeur d'eau par partie en poids d'oligoméres.

4. Procédé suivant les revendications 1 à 3, caractérisé en ce qu'on introduit les oligomères dans un état liquide ou solide dans le lit fluidisé d'oxyde d'aluminium.

5. Procédé suivant les revendications 1 à 4, caractérisé en ce qu'on utilise de l'oxyde d'aluminium γ pour le lit fluidisé d'oxyde d'aluminium.

6. Procédé suivant les revendications 1 à 5, caractérisé en ce qu'on utilise le méthanol en tant qu'alcanol.